(19) Europäisches Patentamt
European Patent Office
Office européen des brevets

(11) EP 2 162 484 B1

(12) **EUROPEAN PATENT SPECIFICATION**

(45) Date of publication and mention of the grant of the patent:
**27.02.2019 Bulletin 2019/09**

(21) Application number: **08778051.6**

(22) Date of filing: **04.07.2008**

(51) Int Cl.:
***C08G 73/10*** (2006.01) ***C07C 229/60*** (2006.01)
***H01L 21/283*** (2006.01) ***H01L 21/288*** (2006.01)
***H01L 21/336*** (2006.01) ***H01L 29/786*** (2006.01)
***H01L 51/30*** (2006.01) ***H01L 27/28*** (2006.01)

(86) International application number:
**PCT/JP2008/062530**

(87) International publication number:
**WO 2009/008491 (15.01.2009 Gazette 2009/03)**

(54) **POLYAMIC ACID**

POLYAMINSÄURE

ACIDE POLYAMIQUE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **06.07.2007 JP 2007178703**

(43) Date of publication of application:
**17.03.2010 Bulletin 2010/11**

(73) Proprietor: **Ricoh Company, Ltd.**
**Tokyo 143-8555 (JP)**

(72) Inventors:
- **TANO, Takanori**
  **Chiba 2600044 (JP)**
- **SUZUKI, Koei**
  **Yokohama-shi**
  **Kanagawa 224-0033 (JP)**
- **TSUDA, Yusuke**
  **Kurume-shi**
  **Fukuoka 830-8555 (JP)**

(74) Representative: **SSM Sandmair**
**Patentanwälte Rechtsanwalt**
**Partnerschaft mbB**
**Joseph-Wild-Straße 20**
**81829 München (DE)**

(56) References cited:
**WO-A1-98/38168** **WO-A1-2005/033248**
**CN-A- 1 375 538** **JP-A- 6 345 869**
**KR-A- 20000 020 048** **US-A- 3 647 805**
**US-A1- 2006 073 350** **US-A1- 2006 149 029**

- **WANG D.H. ET AL.: 'Synthesis of stretchable polyetherimides containing multiple alkyl side chains' POLYMER vol. 48, 24 February 2007, pages 2572 - 2584, XP022043336**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

TECHNICAL FIELD

The present invention relates to a polyamic acid.

BACKGROUND ART

Recently, an organic transistor using an organic semiconductor material has been studied actively. The advantages of use of an organic semiconductor material for a transistor include attainment of flexibility and a large surface area and simplification of a process due to a simple layer structure and the like. Furthermore, extraordinarily cheap production than those of conventional apparatuses for producing a silicon-based semiconductor may be attained by using a printing method or the like for a production method. Moreover, it may be possible to make formation of a thin film or circuit simpler by means of a printing method, spin-coat method, sipping method or the like.

One of parameters indicating the characteristics of such an organic thin film transistor is the on/off ratio of electric current (Ion/Ioff). In regard to an organic thin film transistor, the electric current ($I_{ds}$) flowing between source and drain electrodes in a saturation region is represented by the following equation (1):

$$I_{ds} = \frac{\mu C_{in} W (V_G - V_{TH})^2}{2L} \cdots\cdots (1)$$

, wherein $\mu$ is a field effect mobility; and $C_{in}$ is a capacitance per unit area of a gate insulating film ($C_{in} = \varepsilon\varepsilon_0/d$, wherein $\varepsilon$ is a dielectric constant of the gate insulating film; $\varepsilon_0$ is a dielectric constant of vacuum; and d is a thickness of the gate insulating film); W is a channel width; L is a channel length: $V_G$ is a gate voltage; and $V_{TH}$ is a threshold voltage.

This equation indicates that it is effective (1) to increase the field effect mobility $\mu$, (2) to decrease the channel length L, (3) to increase the channel width W, and the like, in order to increase the on-state current. Because the field effect mobility $\mu$ largely depends on material properties, various kinds of materials have been developed in order to improve the field effect mobility $\mu$. On the other hand, because the channel length L depends on element structures, efforts for improving the on-state current have been conducted based on modification of the element structures. A general method for decreasing the channel length L is to reduce the distance between the source and drain electrodes. The channel length L is required to be generally 10 $\mu$m or less, and preferably 5 $\mu$m or less, because the field effect mobility $\mu$ of an organic semiconductor material is intrinsically not so large.

Meanwhile, one of methods for precisely manufacturing a organic thin film transistor with a small distance between source and drain electrodes is a photolithographic process used in a silicon process, and this process is conducted by the following steps:

(1) forming a photo-resist layer by applying a resist on thin film layers laminated on a substrate (a resist application process);
(2) removing a solvent in the photo-resist layer by means of heating (a pre-bake process);
(3) exposing the solvent-removed photo-resist layer to ultraviolet rays through a hard mask patterned by using a laser or electron beams in accordance with pattern data (an light exposure process);
(4) removing a light-exposed resist by treating the ultraviolet rays-exposed photo-resist layer with an alkaline solution (a development process);
(5) curing the resist on light-unexposed areas (patterned areas) by means of heating (a post-bake process);
(6) removing the thin film layer on the resist-removed area by means of dipping in an etching liquid or exposure to an etching gas (an etching process); and
(7) removing the resist by means of an alkaline solution or an oxygen radical (a resist removal process).

An active element is completed by repeating all the aforementioned processes (1) to (7) after forming each of predetermined thin film layers but expensive equipment and long processes cause cost increase.

Therefore, an electrode pattern formation based on a printing method utilizing an ink-jet method or the like has been studied in order to reduce the cost of production of an organic thin film transistor. Because such ink jet printing is allowed to form an electrode pattern directly, the efficiency of use of a material is high and it may be possible to attain the simplification of a production process and the cost reduction. However, because it is generally difficult to reduce the ejection quantity in the ink-jet printing, it is also difficult to form a pattern with a size of 30 $\mu$m or less and it may be impossible to attain an electrode spacing of about 5 $\mu$m when the landing precision depending on a mechanical error

or the like is taken into consideration. This indicates that it may be difficult to a highly precise device by using only an ink jet apparatus. Therefore, some modification for attaining the high precision is required and it is considered that some modification is applied on an ink-landing surface.

For example, JP-A-2005-310962 may suggest a method utilizing a gate insulating film made of a material whose critical surface tension (also referred to as a surface free energy) can be changed by means of application of energy such as that of ultraviolet rays. This method is to produce a high surface free energy area on the surface of an insulating film by irradiation of only an electrode making area with ultraviolet rays while utilizing a mask. When an electrode material made of a water-soluble ink is ink-jet-applied thereon, an electrode is formed only on the high energy area and therefore it becomes possible to form a highly precise electrode pattern on a gate insulating film. In a case of use of this method, although an ink drop lands on the borderline between a high surface free energy area and a low surface free energy area, the ink drop moves to the side of the high surface free energy area due to the difference between the surface free energies, and accordingly, it may be possible to manufacture a pattern having a uniform line.

Furthermore, the Proceedings of the 52th Meeting of the Japan Society of Applied Physics and Related Societies, p. 1510, may suggests a method laminating, on a gate insulating film, a film made of a material whose surface free energy can be changed by means of ultraviolet rays. On the laminated film, areas with surface free energies different from one another are formed by irradiation with ultraviolet rays similarly to the method as disclosed in JP-A-2005-310962 and a highly precise electrode pattern is formed by an ink-jet method.

Herein, the method as disclosed in JP-A-2005-310962 may be excellent in that it may be possible to attain an electrode spacing of 5 $\mu$m or less but the light sensitivity of a material used for forming a high surface free energy area is low, and accordingly, irradiation with ultraviolet rays with a high energy of 10 J/cm$^2$ or greater, inter alia, ultraviolet rays with a short wavelength of 300 nm or less, is required. This indicates that a long irradiation time period is required even if a high power ultraviolet lamp is used. As a result, the tact time is long and there is a problem that simplification of a production process or cost reduction is not sufficient even though an inexpensive printing process such as an ink jet method is used.

Furthermore, sufficient simplification of a production process is not expected in the method as disclosed in the Proceedings of the 52th Meeting of the Japan Society of Applied Physics and Related Societies, p. 1510, because a laminated structure is formed. Moreover, the light sensitivity of a material for forming a high surface energy area is also low similarly to as disclosed in JP-A-2005-310962, and as disclosed in the Proceedings of the 52th Meeting of the Japan Society of Applied Physics and Related Societies, p. 1510, irradiation with ultraviolet rays with 20 J/cm$^2$ or greater, inter alia, ultraviolet rays with a short wavelength of 300 nm or less, is required. This indicates that a longer irradiation time period is required even if a high power ultraviolet lamp is used. As a result, the tact time is long and there is a problem that simplification of a production process or cost reduction is not expected even though an inexpensive printing process such as an ink jet method is used.

Thus, a method for manufacturing a high surface free energy area and a low surface energy area on a gate insulating film by means of ultraviolet rays or electron beams may make it possible to form a highly precise and dense electrode pattern, which has been difficult by a conventional printing method. However, the light sensitivity of a material whose surface free energy can be changed by ultraviolet rays is low and it is necessary to conduct irradiation with high energy light for a long time period. Accordingly, there occurs a problem that simplification of a production process or cost reduction is not expected. Herein, the inventors have found that it is required to provide a material which can reduce the energy of irradiation.

Thus, the inventors have also found that it is desired to provide a material capable of reducing a time period for irradiation with high energy light, that is, of changing the surface free energy of a film by means of a less irradiation energy. Furthermore, the inventors have also found that it is desired to provide an electronic element, electronic element array and display device using such an electrode layer because the inventors have found that an electrode layer could be formed inexpensively for a short tact time.

JP 06-345869 A relates to a polyimide and its production. In order to easily and surely obtain a polyimide having high heat resistance and mechanical strength and low thermal expansion coefficient a specific aromatic diamine component is reacted with a specific aromatic acid dianhydride component. In this respect, the objective polyimide containing the recurring unit of formula III as a main recurring unit is produced by reacting (A) di(4-aminophenyl)-1,4-benzenecarboxylic acid dianilide of formula I as an aromatic diamine component with (B) an aromatic acid dianhydride of formula II (R is tetravalent organic group) as an aromatic acid dianhydride component and dehydrating the obtained polyamic acid.

Formula I:

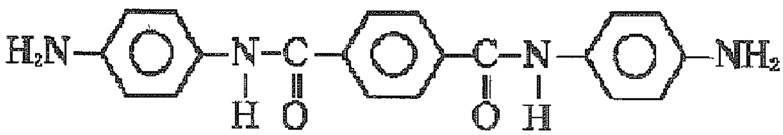

Formula II:

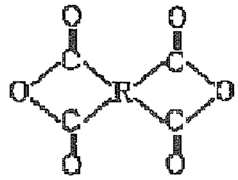

Formula III:

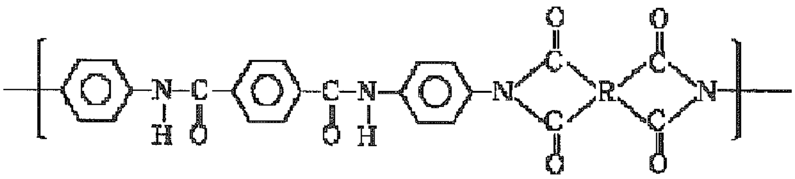

US 2006/0149029 A1 discloses a functional diamine compound having a dendron structure, polyamic acid which is produced using functional diamine, aromatic cyclic diamine, aliphatic cyclic acid dianhydride, and aromatic cyclic acid dianhydride, polyimide which is produced by imidizing polyamic acid, and an LC alignment film produced using polyimide. Even if the diamine compound is used in a small amount, it is possible to realize a high pretilt angle, thus the pretilt angle is easily controlled. Therefore, it can be used to produce an LC alignment film using a twisted nematic (TN) mode, in which the pretilt angle of liquid crystal is low, and a vertically aligned (VA) mode, which requires a high pretilt angle of about 90°.

US 2006/0073350 A1 discloses a diamine compound having a dendron side chain and a liquid crystal alignment material produced using the diamine compound. Specifically, the diamine compound is used to prepare a polyamic acid, which is then used to produce the liquid crystal alignment material. According to the liquid crystal alignment material, the pretilt angle of a liquid crystal is easy to control, and the alignment properties of a liquid crystal are good. Particularly, since the liquid crystal alignment material shows superior chemical resistance to washing solvents used in LCD panel fabrication processes, it has an advantage in that the alignment properties of a liquid crystal are not degraded even after washing.

CN 1 375 538 A and KR 2000 0020048 A describe technological background.

DISCLOSURE OF THE INVENTION

The scope of the invention is defined in the appended claims. Any reference to "embodiment(s)", "example(s)" or "aspect(s) of the invention" in this description not falling under the scope of the claims should be interpreted as illustrative example(s) for understanding the invention.

According to one aspect not forming part of the present invention, there is provided a diamine compound represented by the following formula (1):

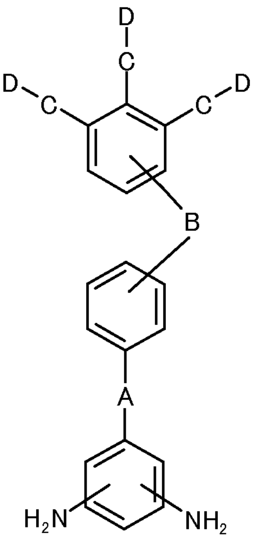

(1)

,

wherein each of group A and group B is -O-, -COO-, - CONH-, or -OCO-; groups C are each independently -O-,-COO-, -CONH-, or -OCO-; and groups D are each independently a C1 to C20 linear, branched, or cyclic alkyl group unsubstituted or substituted with at least one halogen atom, or a functional group represented by the following formula (2):

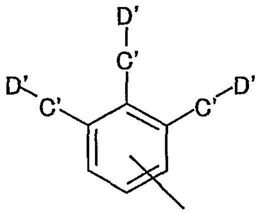

(2)

,

wherein substituents C' are each independently -O-,-COO-, -CONH-, or -OCO-; and substituents D' are each independently a C1 to C20 linear, branched, or cyclic alkyl group or a functional group represented by the following formula (3):

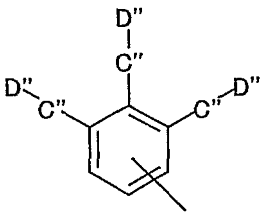

(3)

,

wherein substituents C" are each independently -O-,-COO-, -CONH-, or -OCO-; and substituents D" are each independently a C1 to C20 linear, branched or cyclic alkyl group or a functional group represented by the following formula (4):

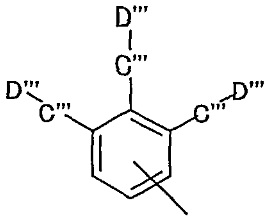

(4)

,

wherein substituents C"' are each independently -O-,-COO-, -CONH-, or -OCO-; and substituents D"' are each independently a C1 to C20 linear, branched, or cyclic alkyl group.

According to the present invention, there is provided a polyamic acid obtained by copolymerizing monomers

comprising the diamine compound as described above and at least one of an alicyclic acid dianhydride and aromatic acid dianhydride, wherein the diamine compound is represented by formula (5) or (6) of claim 1.

According to an aspect not forming part of the present invention, there is provided a soluble polyimide obtained by imidating a part(s) or all of an amide group(s) of the polyamic acid as described above.

According to another aspect not forming part of the present invention, there is provided a composition comprising the polyamic acid as described above and the soluble polyimide obtained by imidating a part(s) or all of an amide group(s) of the polyamic acid as described above.

According to another aspect not forming part of the present invention, there is provided a wettability changing film obtained by applying the soluble polyimide as described above on a substrate.

According to another aspect not forming part of the present invention, there is provided a wettability changing film obtained by applying the composition as described above on a substrate.

According to another aspect not forming part of the present invention, there is provided an electrode comprising the wettability changing film as described above.

According to another aspect not forming part of the present invention, there is provided a method of manufacturing a wettability changing film, comprising the steps of dissolving the polyamic acid as described above in a solvent to obtain a solution, applying the solution on a substrate, and imidating a part(s) or all of the solution.

According to another aspect not forming part of the present invention, there is provided a method of manufacturing a wettability changing film, comprising the steps of dissolving the soluble polyimide as described above in a solvent to obtain a solution, applying the solution on a substrate, and imidating a part(s) or all of the solution.

According to another aspect not forming part of the present invention, there is provided a method of manufacturing a wettability changing film, comprising the steps of dissolving the composition as described above in a solvent to obtain a solution, applying the solution on a substrate, and imidating a part(s) or all of the solution.

## BRIEF DESCRIPTION OF THE DRAWINGS

FIG. 1 is a schematic diagram showing an organic transistor in which a wettability changing film layer is laminated on a highly insulating layer.

FIGS. 2A and 2B show one example of an electronic element array, wherein FIG. 2A is a cross-sectional view thereof and FIG. 2B is a plan view the configuration of electrodes, etc.

FIG. 3 is a cross-sectional diagram showing a display device using the electronic element array of FIGS. 2A and 2B.

FIG. 4 is a schematic diagram showing a digital paper sheet in which the display device of FIG. 3 is used as a display medium.

FIG. 5 is a diagram showing an apparatus in which the display device of FIG. 3 is installed in a display medium such as a pocket-sized PC.

FIG. 6 is a $^1$H-NMR spectrum of a diamine (17) prepared in preparation example 1 in an embodiment of the present invention.

FIG. 7 is an FT-IR spectrum of the diamine (17) prepared in preparation example 1 of the embodiment of the present invention.

FIG. 8 is a thermogram of the diamine (17) prepared in preparation example 1 of the embodiment of the present invention which thermogram was obtained by means of a differential scanning calorimetry (DSC).

## EXPLANATION OF LETTERS OR NUMERALS

1: Highly insulating layer
2: Wettability changing layer
3: Low surface free energy area with a small critical surface tension
4: High surface free energy area with a large critical surface tension
5: Electrically conductive layer
5a: Source electrode layer
5b: Drain electrode layer
6: Semiconductor layer
7: Substrate
41: Electronic element
42: Gate electrode

51: Electronic element array
61: Display device
62: Transparent electrode
63: Polyethylene naphthalate substrate
65: Titanium oxide particle
66: ISOPER colored with oil blue
67: Microcapsule
68: PVA binder

## BEST MODE FOR CARRYING OUT THE INVENTION

In the following, some embodiments and examples of the present invention will be described with reference to the accompanying drawings.

According to a first embodiment not forming part of the present invention, there is provided a diamine compound which is represented by the following formula (1):

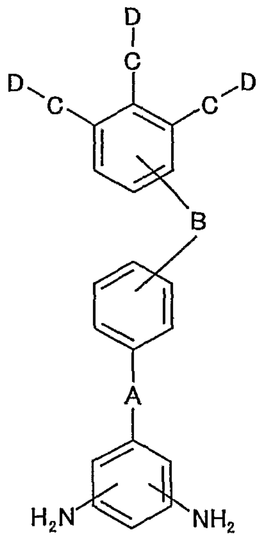

(1)

In the formula, each of group A and group B is -O-, -COO-, -CONH-, or -OCO-, groups C are each independently -O-, -COO-, -CONH-, or -OCO-, and groups D are each independently a C1 - 20 linear, branched, or cyclic alkyl group which may be substituted with at least one halogen atom, or a functional group represented by the following formula (2):

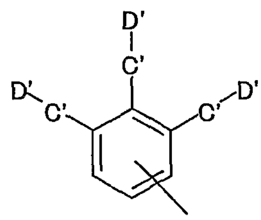

(2)

In the formula, substituents C' are each independently -O-, -COO-, -CONH-, or -OCO-, and substituents D' are each independently a C1 - 20 linear, branched, or cyclic alkyl group or a functional group represented by the following formula (3):

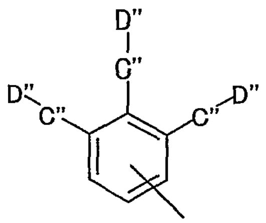

(3)

In the formula, substituents C" are each independently -O-, -COO-, -CONH-, or -OCO-, and substituents D" are each independently a C1 - 20 linear, branched or cyclic alkyl group or a functional group represented by the following formula (4):

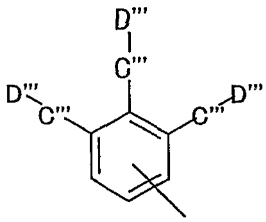

(4)

In the formula, substituents C"' are each independently -O-, -COO-, -CONH-, or -OCO-, and substituents D"' are each independently a C1 - 20 linear, branched, or cyclic alkyl group.

The diamine compound has a dendritic side chain and is represented by the following formula (5):

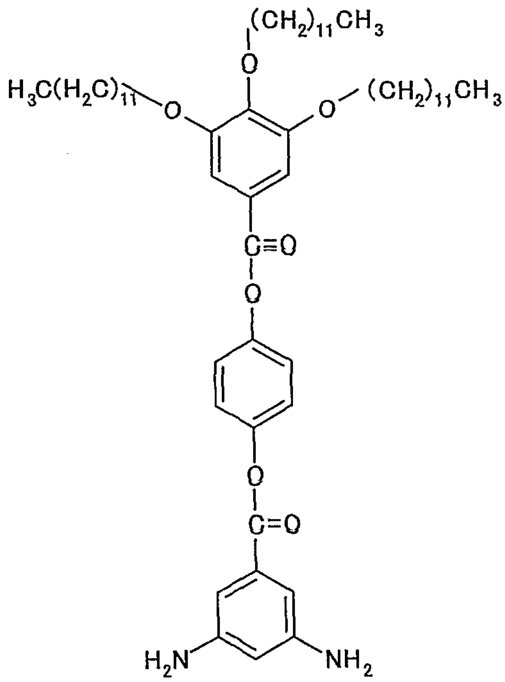

(5)

or formula (6) :

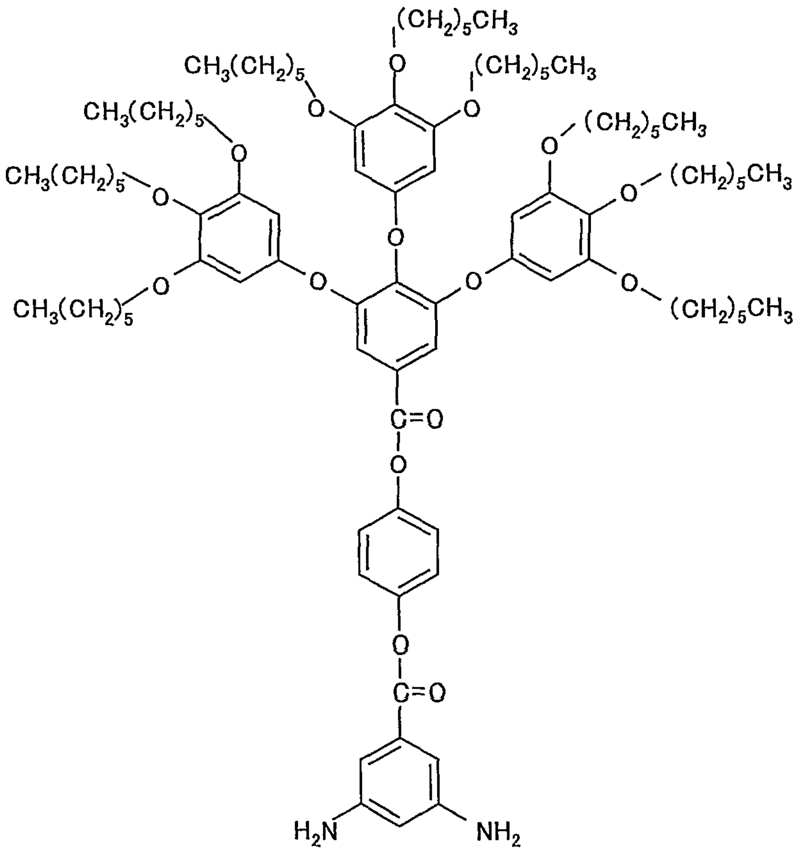

(6)

For the present invention, there is provided a polyamic acid which is obtained by copolymerization of a diamine compound according to an embodiment of the present invention, an alicyclic acid dianhydride and/or an aromatic acid dianhydride wherein the diamine compound is represented by the formula (5) or (6). In addition, there is also provided a polyamic acid that is a copolymer obtained when the above-mentioned monomers further include an aromatic diamine and/or a diaminosiloxane. Such a polyamic acid is prepared by a conventionally known copolymerization method while the diamine compound and the acid dianhydride are used. The copolymerization method is not particularly limited as far as it is usable for preparation of the polyamic acid.

The diamine compound according to the present invention is represented by formula (5) or (6) and has a dendritic structure such that many alkyl groups are introduced to side chains simultaneously, and even if the content of a diamine compound according to the present invention is low, it is possible to provide an organic material which is allowed to provide a highly water-repellent surface (a surface with a low surface free energy).

Meanwhile, a diamine compound according to the present invention (as also referred to as a functional diamine compound) includes a backbone of benzene ring between a diaminophenyl group and a dendritic side chain, which backbone serves as a spacer. A carbonyl group or an atom having a lone pair of electrons adjacent to the backbone of benzene ring increases the spread of the $\pi$-electron conjugation thereof, and as a result, there is provided a molecule that exhibits absorption in a wavelength region of ultraviolet rays which have a relatively long wavelength, for example, about 250 to 300 nm. Thereby, for example, when ultraviolet rays in a wavelength region of 250 to 300 nm are absorbed, the molecule is readily cleaved at this site. A fragment produced after the cleavage reacts with an ambient water or oxygen molecule so as to have a -COOH group or an -OH group and to have a hydrophilic property (to provide a surface with a high surface free energy).

Therefore, even if a film made of a polyamic acid containing a functional diamine monomer according to the present invention as a monomer unit is a film having a water-repellent surface at the time of formation thereof, it is possible to decompose or oxidize the film portion so as to change to a hydrophilic surface by applying energy of electromagnetic waves such as ultraviolet rays and excimer laser or heat onto an arbitrary portion thereof. When the method is used for a surface made of a polyamic acid according to the present invention, it is possible to manufacture a surface having an arbitrary surface free energy by controlling the irradiation energy.

For preparation of a polyamic acid according to an embodiment of the present invention, it is possible to use a diaminosiloxane, for example, a diaminosiloxane having a structure represented by the following formula (7):

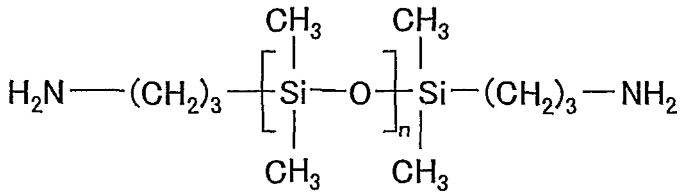 (7)

In the formula, n is an integer of 1 to 10. The content of a functional diamine compound unit as represented by the following formula (a) in a polyamic acid is in a range of 0.1 mole % to 100 mole %, preferably 0.5 mole % to 30 mole %, and more preferably 1 mole % to 30 mole %, based on the total amount of the diamine monomer unit.

The amount of an aromatic diamine and/or diaminosiloxane used in a polyamic acid is in a range of 0 to 99.9 mole %, preferably 70 to 99.5 mole %, and further preferably 70 to 99 mole %, based on the total amount of the diamine monomer.

An aromatic acid dianhydride used for preparation of a polyamic acid according to an embodiment of the present invention is allowed to provide a durable film with a thickness of 1 μm, because it is possible to conduct its copolymerization with the diamine compound such that a resistance to electromagnetic wave or heat energy or a resistance to a chemical substance is provided.

Examples of such an aromatic acid dianhydride include pyromellitic dianhydride (PMDA), biphthalic dianhydride (BPDA), oxydiphthalic dianhydride (ODPA), benzophenone tetracarboxylic dianhydride (BDTA), and hexafluoroisopropylidene diphthalic dianhydride (6-FDA), but it is not limited to them.

A preferred content of an aromatic acid dianhydride is in a range of 10 mole % to 95 mole %, and preferably 50 mole % to 90 mole %, based on the total amount of the acid dianhydride mononer(s). When the content of an aromatic acid dianhydride to be used is less than 10 mole %, the mechanical properties and heat resistance of a manufactured wettability changing film may be poor. On the other hand, when the content of an aromatic acid dianhydride to be used is over 80 mole %, the electric properties may be degraded (for example, the electric leakage may be increased).

An alicyclic acid dianhydride to be used for preparation of a polyamic acid according to an embodiment of the present invention may solve at least one of problems caused by the molecular structure of a polyamic acid and the like, which problems include, for example, insolubility in a general organic solvent, a low transparency in a visible region which is caused by formation of a charge-transfer complex, and low electrotechnical properties caused by the high polarity.

Examples of an aliphatic acid dianhydride include 5-(2,5-dioxotetrahydrofuryl)-3-methylcyclohexene-1,2-dicarboxylic dianhydride (DOCDA), bicyclooctene-2,3,5,6-tetracarboxylic dianhydride (BODA), 1,2,3,4-cyclobutane tetracarboxylic dianhydride (CBDA), 1,2,3,4-cyclopentane tetracarboxylic dianhydride (CPDA), and 1,2,4,5-cyclohexane tetracarboxylic dianhydride (CHDA), but it is not limited to them. The content of an alicyclic acid anhydride is desirably in a range of 5 mole % to 90 mole % and preferably 10 mole % to 50 mole %, based on the total amount of the acid anhydride monomer(s).

A polyamic acid according to an embodiment of the present invention is excellent in its solubility in a general aprotic and polar solvent, for example, N-methyl-2-pyrrolidone (NMP), γ-butyrolactone (GBL), dimethylformamide (DMF), dimethylacetoamide (DMAc), tetrahydrofuran (THF), or the like. It is considered that the excellent solubility of the polyamic acid in the aprotic and polar solvent may significantly influence the steric effect of the funcation diamime in a three-dimensional structure which effect is caused by introduction of the alicyclic acid anhydride and a large steric hindrance among three or more benzene rings. A better solvent provides a positive effect on the printing suitability of a polyamic acid film when it is formed on a substrate.

The number average molecular weight of the polyamic acid according to an embodiment of the present invention is preferably 10,000 to 500,000. When the polycmic acid is imidated, the imidated polyamic acid has a glass transition temperature of 200 to 350 °C depending on the imidation rate and the structure of the polyamic acid.

A third embodiment not forming part of the present invention is an imidated polyimide obtained by means of heat treatment of the polyamic acid according to an embodiment of the present invention or the like. The polyimide may be a product obtained by imidating a part(s) or all of an amide group(s) of a polyamic acid according to an embodiment of the present invention. Herein, a polyimide soluble in a solvent, in particular, the above-mentioned aprotic and polar solvent is preferable. Because the polyimide is an imidated polyamic acid according to an embodiment of the present invention, the composition and molecular weight of the monomer unit are substantially the same as those of a polyamic acid according to an embodiment of the present invention.

Furthermore, a fourth not forming part of the present invention is a composition that is a mixture of the above-mentioned polyimide and the above-mentioned polyamic acid. It is possible to use the polyimide according to the third embodiment and the composition according to the fourth embodiment for a similar application to that of the above-mentioned polyamic acid because it is possible to control the surface free energy easily by means of ultraviolet ray irradiation or the like similarly to the polyamic acid according to the first and second embodiments.

A fifth embodiment not forming part of the present invention is a wettability changing film that is a film obtained by dissolving the polyamic acid according to an embodiment of the present invention in a solvent, coating a substrate with an obtained solution, and imidating the coated solution entirely or partially. The wettability changing film may also be manufactured by directly coating a substrate with the polyimide or composition according to an embodiment of the present invention.

The wettability changing film is a material that is easy to control the uniformity of a hydrophilic surface or water-repellent surface and the contact angle in a range of 0° to 40° for an electrode material in practical examples as described below.

According to an embodiment not forming part of the present invention, a film surface may be provided which is used for manufacturing an electrode of an organic thin film transistor or the like by means of small energy, because a functional diamine compound having a dentritic side chain is easy to modify the surface free energy by means of energy application. FIG. 1 shows a schematic diagram of an organic transistor in which a wettability changing film layer is laminated on a highly insulating film layer. Herein, the highly insulating film layer and the wettability changing film layer are laminated so as to function as a gate insulating film. Source and drain electrodes are manufactured by utilizing a change of the surface free energy of a wettability changing film. Furthermore, it is easily conceivable that it is possible to prepare a wettability changing film between a substrate and a gate electrode and to manufacture the gate electrode by utilizing a change of the surface free energy (not shown in the figure).

FIGS. 2A and 2B show one example of an electronic element array using an organic transistor as shown in FIG. 1. FIG. 2A is the cross-sectional view and FIG. 2B is a plan view of the arrangement of electrodes and the like. It is easily conceivable that it is possible to manufacture an outlet of leads for the electrodes of the electronic element array by using a wettability changing film manufactured by a material according to an embodiment of the present invention (not shown in the figures).

FIG. 3 is a cross-sectional view showing a display device using an electronic element array as shown in FIGS. 2A and 2B. Herein, an electrophoretic display device is shown in which ISOPER colored with oil blue and a titanium oxide particle are encapsulated. The display device may not be electrophoretic and may be, for example, a polymer-dispersion-type liquid crystal device. Furthermore, a color material for the display may have any color.

FIG. 4 is a schematic diagram showing a digital paper sheet in which a display device as shown in FIG. 3 is used as a display medium. As shown in FIG. 5, it is also possible to install and use such a display medium in a computer such as a pocket-sized PC or the like. In addition, FIGS. 4 and 5 are merely schematic diagrams illustrating one example of an image display device or apparatus which may be used for a display medium of a copying machine, a display medium embedded in a seat item, a front glass face or the like of a mobile transportation medium such as an automobile and an airplane, a tag displaying a price in a supermarket, and the like.

Meanwhile, it is inferred that a polyimide film synthesized by using a functional diamine compound having a dendritic side chain according to an embodiment of the present invention readily has a structure such that a side chain is oriented toward ambient air (a direction except directions in the film surface), because such a water-repellent side chain (hydrocarbon chain or halogen chain) projects from the main chain. Therefore, it is considered that liquid crystal molecules contacting the film may be readily oriented, even oriented perpendicularly, by means of rubbing or the like. That is, it is considered that it has a high potential ability to be used for a liquid crystal orientation film (perpendicular orientation film). According to an embodiment of the present invention, the functional diamine compound having a dendritic side chain may also provide a film surface capable of modifying the surface free energy by means of energy application. Therefore, for example, it may also be used for a polyimide bank of an organic electroluminescent device (organic EL device) or the like.

(Practical Examples)

The embodiments of the present invention will be described in more detail with reference to the following practical examples. Herein, the practical examples are provided by way of illustration and the scope of the present invention is not limited in any way.

(1) Preparation of acid chloride (12)

An acid chloride (12) was prepared in accordance with the following reaction scheme:

(8) (9) (10)

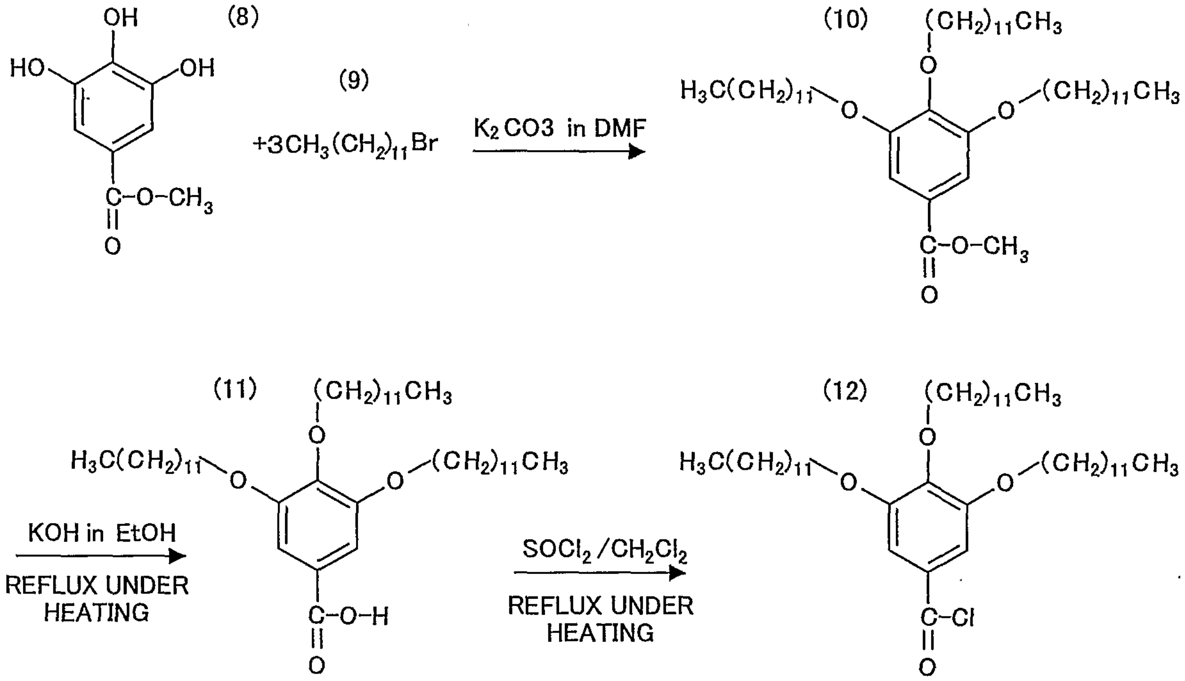


(11) (12)

1 mole of a substituted benzoic acid ester (8) and 8.97 mole of potassium carbonate were added into a solvent, dimethylformamide (DMF) in a round bottom flask to which a condenser was attached, which were stirred sufficiently. After 3 mole of bromododecane (9) was added into the solution, the reaction temperature of the flask was raised slowly up to 70 °C. Afterward, the reaction was continued for 24 hours while the reaction mixture was kept at 70 °C. After completion of the reaction, the temperature was lowered to room temperature. The reaction solution was poured into deionized water so as to yield a precipitate. The precipitated was filtered and washing was repeated so as to obtain a dodecane-substituted product (10). The obtained dodecane-substituted product (10) was dissolved in ethanol and subsequently potassium hydroxide was added into it. The ethanol solution was refluxed for 4 hours so as to obtain an acid derivative (11). The acid derivative (11) was refluxed with thionyl chloride and dichloromethane for 4 hours, so as to obtain an acid chloride (12) through chlorination.

(2) Preparation of diamine (17)

A dimaine (17) was prepared in accordance with the following reaction scheme:

(12) (13) (14)

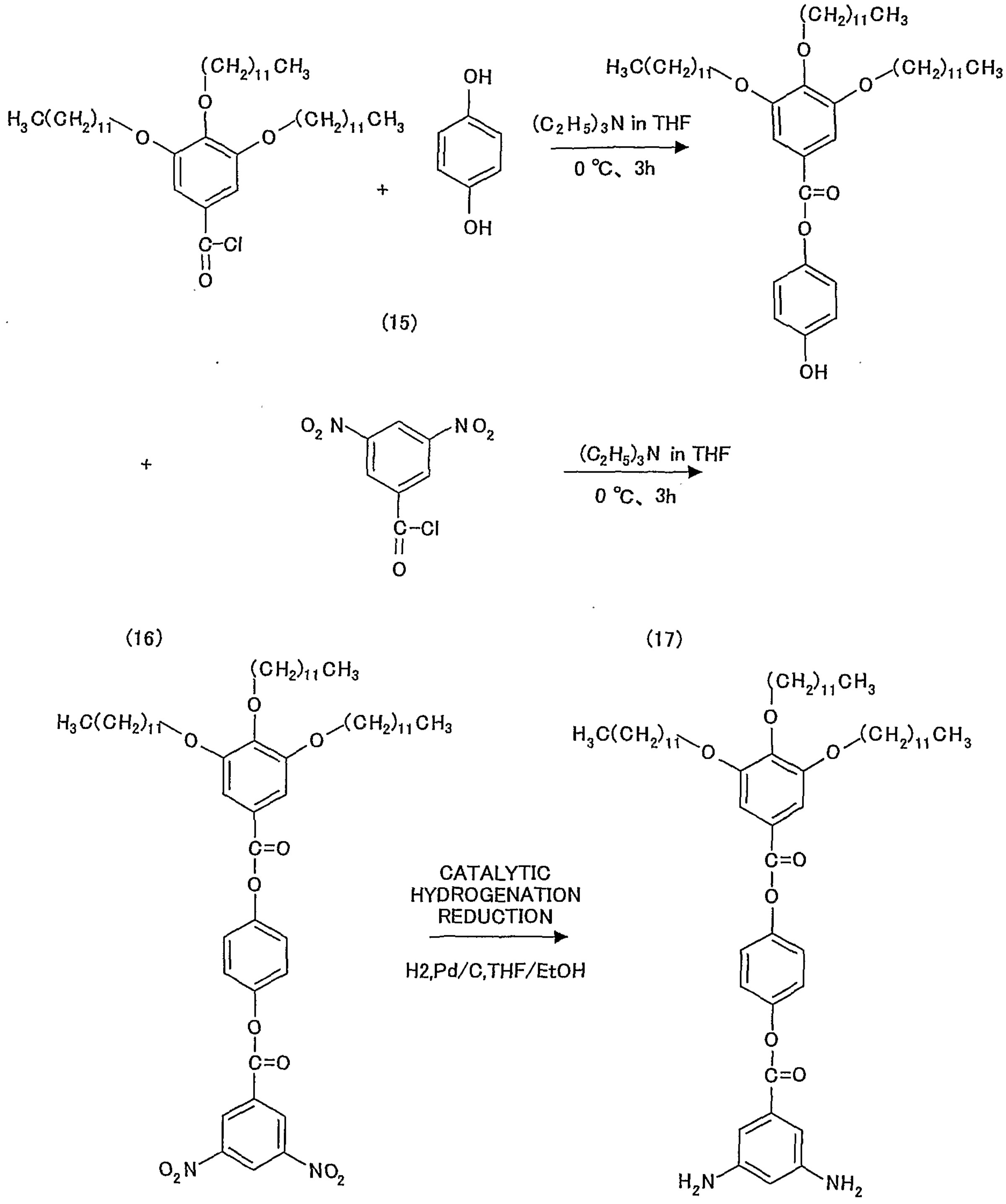


(15)

(16) (17)

In a sufficiently dried round bottom flask, 1 mole of hydroquinone (13) and triethylamine as a catalyst were dissolved in THF. 1 mole of the prepared acid chloride (12) was dropped into the solution slowly and it was cooled in an ice bath and stirred for 3 hours so as to yield an intermediate (14). The intermediate (14) was dissolved in THF and triethylamine as a catalyst was added into it. 1 mole of 3,5-dinitrobenzoyl chloride (15) was added into the solution. The obtained mixture was subjected to reaction at room temperature for 3 hours so as to yield a dinitro compound (16). Then, moisture and oxygen present in a sufficiently washed and dried round bottom flask were removed under vacuum, and instead, the flask was filled with argon (Ar) as an inert gas. After the dinitro compound (16) was thrown into the flask, a mixed solvent of ethanol and THF were added so as to dissolve the dinitro compound (16). Palladium - carbon as a

catalyst was added and catalytic hydrogenation reduction was conducted at 0.3 MPa and room temperature for 5 hours. After completion of the reaction, a reaction product was purified by means of column chromatography and recrystallization so as to obtain a diamine (17) as a final product.

The structure of the obtained diamine (17) was characterized based on its 1H-NMR spectrum and FT-IR spectrum and its thermal property was evaluated by means of differential scanning calorimetry (DSC). These results were shown in FIGS. 6, 7 and 8.

(Practical example 1)

0.95 mole of 4,4'-methylenedianiline (18):

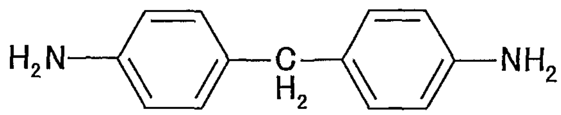

(18)

and 0.05 mole of the prepared dimaine (17) were thrown into a four-necked flask to which a stirrer, a thermostat, a nitrogen injector, and a condenser were attached, while nitrogen was injected. Then, a solution of N-methyl-2-pyrrolidone (NMP) was added into it and an obtained mixture was dissolved sufficiently. 0.5 mole of 5-(2,5-dioxotetrahydrofuryl)-3-methylcyclohexane-1,2-dicarboxylic dianhydride (DOCDA) (19) :

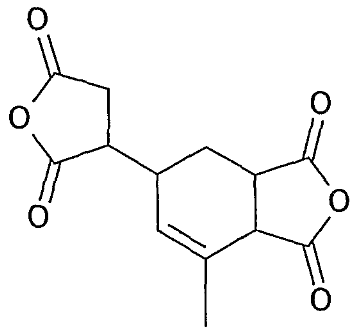

(19)

in the solid state and 0.5 mole of pyromellitic dianhydride (20):

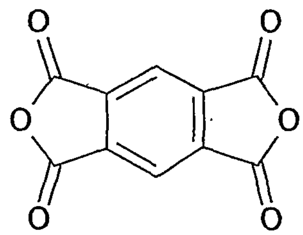

(20)

were added into the solution. The obtained mixture was stirred strongly. At this time, the solid content was 15 % by weight. While the reaction mixture was kept at a temperature less than 25 °C, it was subjected to reaction for 24 hours so as to prepare a polyamic acid solution.

The polyamic acid solution was applied on a glass substrate (10 cm × 10 cm) by means of spin-coating such that its thickness was 100 nm, and was cured at 80 °C and 210 °C so as to manufacture a wettability changing thin film (A). The wettability changing thin film (A) was irradiated with ultraviolet rays (from a high pressure mercury lamp). Changes of the contact angle of a solution containing an electrode material (wherein an aqueous solution containing dispersed silver nano-particles was used and is referred to as a silver nano ink below) was obtained with respect to irradiation time periods by a drop method. The results of changes of the contact angle of the solution containing an electrode material are shown in TABLE 1.

TABLE 1

| | | | | | | |
|---|---|---|---|---|---|---|
| | IRRADIANCE OF ULTRAVIOLET RAYS ( $J/CM^2$ ) | 0 | 2 | 5 | 10 | 15 |
| WETTABILITY CHANGING FILM (A) | CONTACT ANGLE OF ELECTRODE MATERIAL (°) | 34 | 14 | 4 | 4 | 4 |
| | ELECTRODE PATTERNING PROPERTY | D | B | A | A | A |
| WETTABILITY CHANGING FILM (B) | CONTACT ANGLE OF ELECTRODE MATERIAL (°) | 29 | 29 | 27 | 26 | 25 |
| | ELECTRODE PATTERNING PROPERTY | D | D | D | D | D |
| WETTABILITY CHANGING FILM (C) | CONTACT ANGLE OF ELECTRODE MATERIAL (°) | 35 | 24 | 9 | 4 | 4 |
| | ELECTRODE PATTERNING PROPERTY | D | D | B | A | A |

(Comparative example 1)

A polyamic acid was prepared by a method similar to that of practical example 1 except that 0.80 mole of 4,4'-methylenedianiline (18) and 0.20 mole of 2,4-diaminophenoxyhexadecane (21):

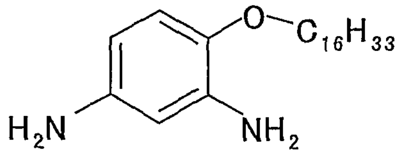

(21)

were used instead of 0.95 mole of 4,4'-methylenedianiline (18) and 0.05 mole of the diamine (17). According to the procedure as described in practical example 1, a wettability changing thin film (B) was manufactured and its contact angles of an electrode material were measured similarly. The results are shown in TABLE 1.

(Comparative example 2)

A polyamic acid was prepared by a method similar to that of practical example 1 except that 0.95 mole of 4,4'-methylenedianiline (18) and 0.05 mole of a diamine compound (22):

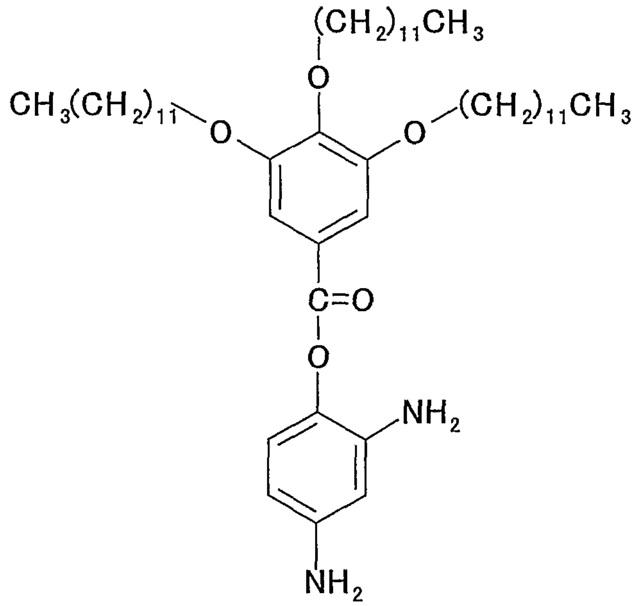

(22)

were used instead of 0.95 mole of 4,4'-methylenedianiline (18) and 0.05 mole of the dianmine (17). According to the procedure as described in practical example 1, a wettability changing thin film (C) was manufactured and its contact angles of an electrode material were measured similarly. The results are shown in TABLE 1.

(Practical example 2)

Next, the electrode patterning properties were compared. The wettability changing thin film (A) manufactured in practical example 1 was irradiated with ultraviolet rays (from a high pressure mercury lamp) through a line-shaped photo-mask such that their irradiance was 1 $J/cm^2$ to 15 $J/cm^2$, whereby a site having a high surface free energy was formed on the thin film. The nano-silver electrode material ink was ejected onto the formed site having a high surface free energy by an ink jet method. After baking in an oven at 210 °C, observation by means of metallographical microscopy was made to confirm whether lines spaced 5 μm apart were formed. The results are shown in TABLE 1.

In regard to the electrode patterning property shown in TABLE 1, D indicates that no lines spaced 5 μm apart were formed, C indicates that some lines spaced 5 μm apart were formed, B indicates that some lines spaced 5 μm apart were not formed, and A indicates that all lines spaced 5 μm apart were formed.

(Comparative example 3)

The wettability changing thin film (B) manufactured in comparative example 1 was irradiated with ultraviolet rays similarly to practical example 2 and the nano silver ink was ejected by an ink jet method. After baking, observation by means of metallographical microscopy was made to confirm whether lines spaced 5 μm apart were formed. The results are shown in TABLE 1.

(Comparative example 4)

The wettability changing thin film (C) manufactured in comparative example 2 was irradiated with ultraviolet rays similarly to practical example 2 and the nano silver ink was ejected by an ink jet method. After baking, observation by means of metallographical microscopy was made to confirm whether lines spaced 5 μm apart were formed. The results are shown in TABLE 1.

These results well correspond to the results of the contact angles of an electrode material with respect to the irradiance of ultraviolet rays, which were obtained in practical example 1. That is, in regard to the wettability changing thin film (A) made from the diamine (17), which is according to the present invention, a surface with a high surface free energy could be formed even if the irradiance of ultraviolet rays was as small as 2 $J/cm^2$. Therefore, if a hydrophilic electrode material (with a high surface free energy) is applied thereon by an ink jet method, a sharp borderline between a surface with a high surface free energy and a surface with low surface free energy should be formed. Furthermore, the wet ink should not spread except the surface with a high surface free energy so that lines spaced 5 μm apart can be formed. On the other hand, in regard to the wettability changing thin film (B) made from the diamine (21), the changes of the contact angle which was caused by ultraviolet rays was as small as 5° at 15 $J/cm_2$ or less as employed in the above-mentioned experiment and no lines spaced 5 μm apart were formed.

Furthermore, although the wettability changing thin film (C) was made from the diamine (22) having a side chain with a dendritic structure similarly to the wettability changing thin film (A) made from the diamine (17), no surface with a high energy was formed unless the irradiance was 5 $J/cm^2$ or greater, and no lines spaced 5 μm apart were partially formed. It is considered that the reason why the irradiance of ultraviolet rays was needed which was higher than that of the wettability changing thin film (A) made from the diamine (17) is that the spread of π-conjugation was small.

Thus, it is found that it is possible to form an electrode by means of a relatively smaller irradiance of ultraviolet rays with respect to a material having a side chain with a dendritic structure and including plural sites absorbing ultraviolet rays with a long wavelength.

According to the present invention, it is possible to provide a polyamic acid that is an organic semiconductor material as a raw material (for a substrate) which is capable of changing a surface free energy of a film by means of an irradiation ray(s) with a small amount of irradiation energy, a composition comprising it and a soluble polyimide, and a diamine compound that is a raw material for its polymerization.

## INDUSTRIAL APPLICABILITY

The present invention may be applied to at least one of a diamine compound, polyamic acid, soluble polyimide, and a composition thereof, and wettability changing film obtained therefrom and electrode, and method of manufacturing a wettability changing film.

Furthermore, the present invention may also be applied to one or both of a diamine compound having a dendritic side chain and a wettability changing material manufactured by using a copolymer of the diamine compound.

Moreover, the present invention may also be applied to a technique for providing a polyamic acid or polyimide which is capable of changing a surface free energy thereof more easily by means of energy application due to introduction of a dendritic side chain into a diamine compound. Herein, it may be relatively easy to control a value of the surface free

energy of the polyamic acid or polyimide and it may be possible to use such a thin film for manufacturing an electrode of an organic thin film transistor or the like.

## Claims

**1.** A polyamic acid obtained by copolymerizing monomers comprising a diamine compound and at least one of an alicyclic acid dianhydride and aromatic acid dianhydride, wherein the diamine compound is represented by the following formula (5) or (6):

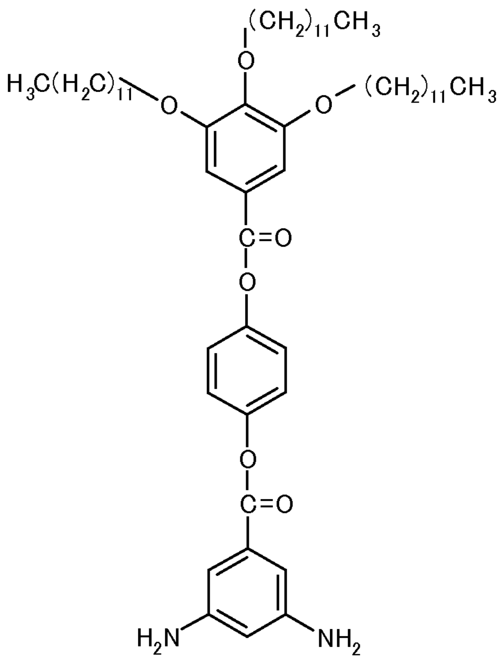

(5)

,

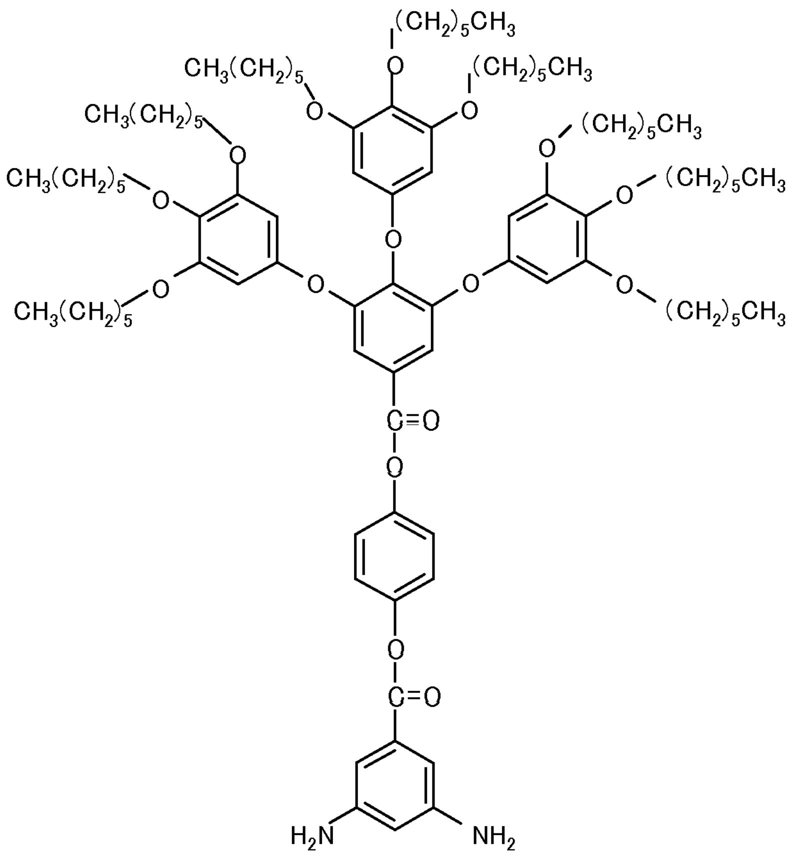

(6)

2. The polyamic acid as claimed in claim 1, wherein the monomers further comprise at least one of an aromatic diamine and diaminosiloxane, wherein an amount of the diamine compound is 0.1 mole % or greater and 100 mole % or less and an amount of the at least one of an aromatic diamine and diaminosiloxane is greater than 0 mole % and 99.9 mole % or less, based on a total amount of the monomers.

3. The polyamic acid as claimed in claim 1, wherein an amount of the alicyclic acid dianhydride is 5 mole % or greater and 90 mole % or less and an amount of the aromatic acid dianhydride is 10 mole % or greater and 95 mole % or less, based on a total amount of the alicyclic acid dianhydride and the aromatic acid dianhydride.

4. The polyamic acid as claimed in claim 1, wherein a number average molecular weight of the polyamic acid is 10,000 or greater and 500,000 or less.

**Patentansprüche**

1. Polyamidsäure, die durch Copolymerisieren von Monomeren erhalten wird, die eine Diaminverbindung und mindestens eines von einem alicyclischen Säuredianhydrid und einem aromatischen Säuredianhydrid umfassen, wobei die Diaminverbindung durch die folgenden Formel (5) oder (6) dargestellt ist:

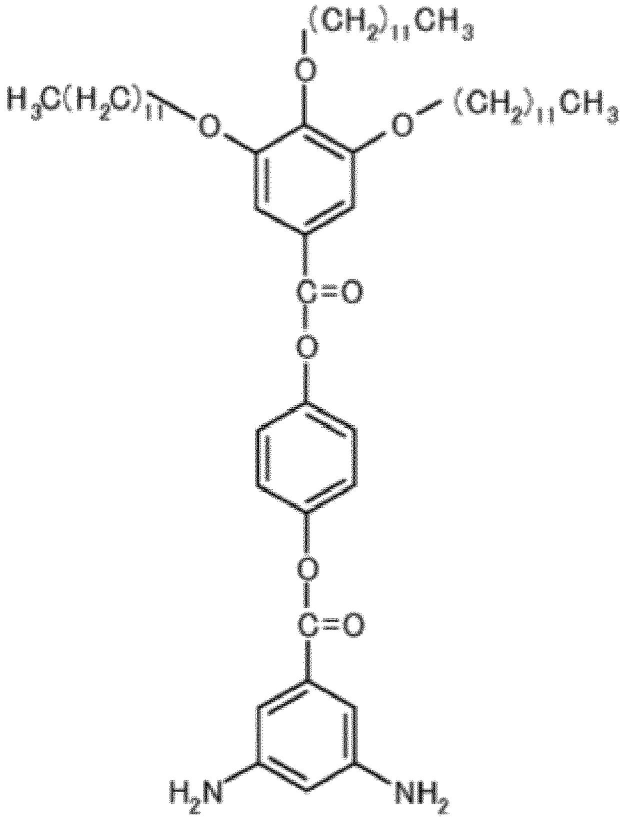

(5)

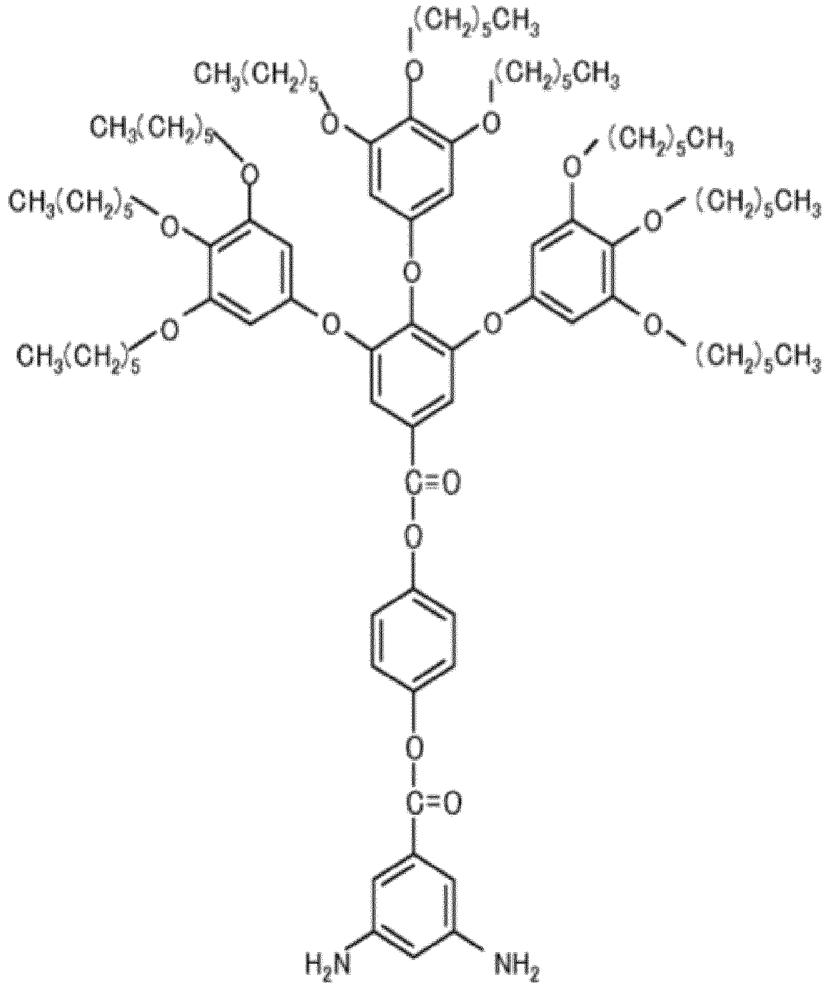

(6)

2. Polyamidsäure nach Anspruch 1, wobei die Monomere ferner mindestens eines von einem aromatischen Diamin und Diaminosiloxan umfassen,
wobei auf Grundlage einer Gesamtmenge der Monomere eine Menge der Diaminverbindung 0,1 % Mol-% oder größer und 100 Mol-% oder weniger ist und eine Menge des mindestens einen von einem aromatischen Diamin und Diaminosiloxan größer als 0 Mol-% und 99,9 Mol-% oder weniger ist.

3. Polyamidsäure nach Anspruch 1,
wobei auf Grundlage einer Gesamtmenge des alicyclischen Säuredianhydrids und des aromatischen Säuredianhydrids eine Menge des alicyclischen Säuredianhydrids 5 Mol-% oder größer und 90 Mol-% oder weniger ist und eine Menge des aromatischen Säuredianhydrids 10 Mol-% oder größer und 95 Mol-% oder weniger ist.

4. Polyamidsäure nach Anspruch 1,
wobei ein zahlenmittleres Molekulargewicht der Polyamidsäure 10.000 oder größer und 500.000 oder weniger ist.

## Revendications

1. Acide polyamide obtenu par la copolymérisation de monomères comprenant un composé de diamine et un dianhydride d'acide alicyclique et un dianhydride d'acide aromatique, le composé de diamine étant représenté par la formule suivante (5) ou (6) :

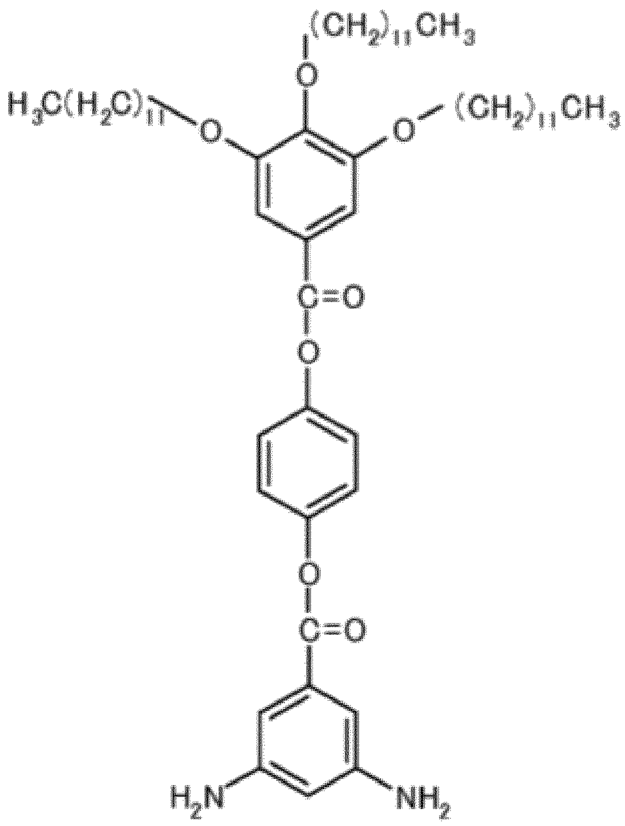

(5)

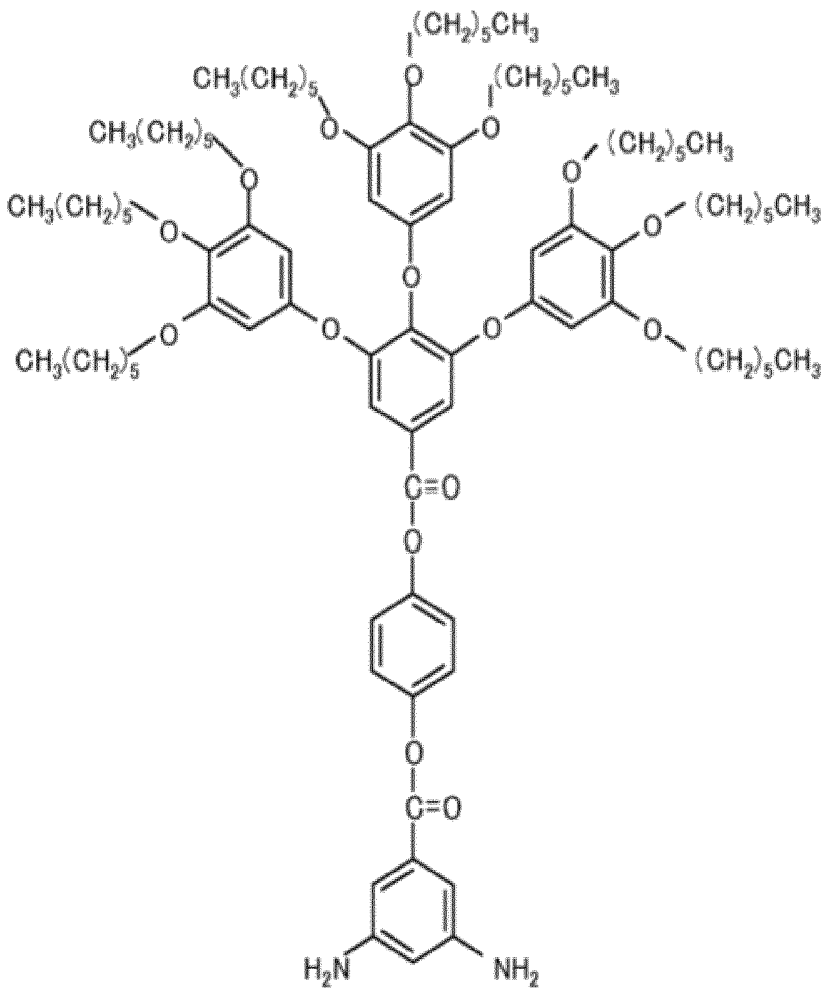

(6)

2. Acide polyamide tel que défini dans la revendication 1, dans lequel les monomères comprennent en outre une diamine aromatique et/ou un diaminosiloxane,
dans lequel une quantité du composé de diamine est de 0,1 % en mole ou supérieure et 100 % en mole ou moins et une quantité de ladite diamine aromatique et/ou dudit diaminosiloxane est supérieure à 0 % en mole et 99,9 % en mole ou moins, en fonction d'une quantité totale de monomères.

3. Acide polyamide tel que défini dans la revendication 1,
dans lequel une quantité de dianhydride d'acide alicyclique est de 5 % en mole ou supérieure et de 90 % en mole ou moins et une quantité de dianhydride d'acide aromatique est de 10 % en mole ou supérieure et de 95 % en mole ou moins, en fonction d'une quantité totale de dianhydride d'acide alicyclique et de dianhydride d'acide aromatique.

4. Acide polyamide tel que défini dans la revendication 1,
dans lequel un poids moléculaire moyen en nombre de l'acide polyamide est de 10 000 ou supérieur et de 500 000 ou moins.

FIG.1

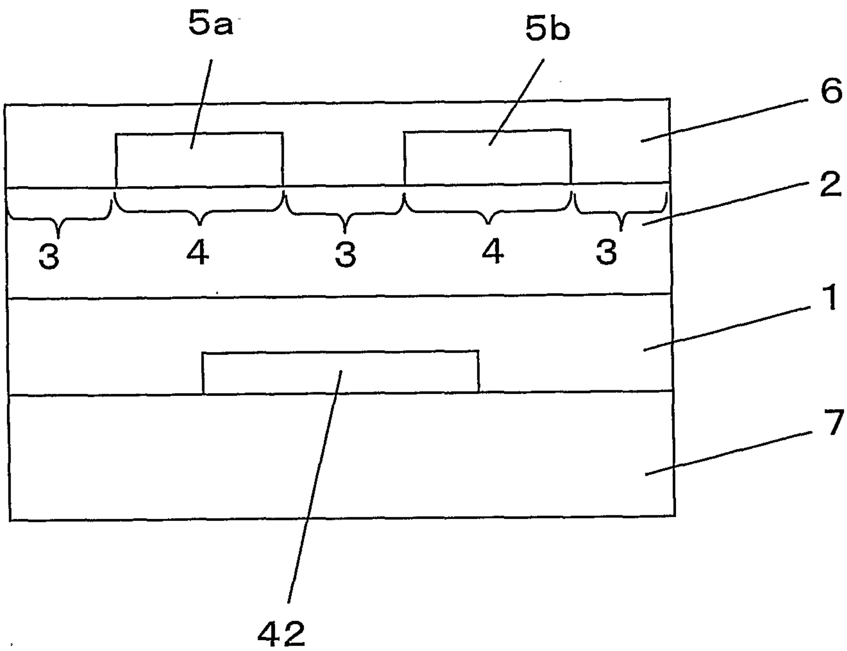

FIG.2A

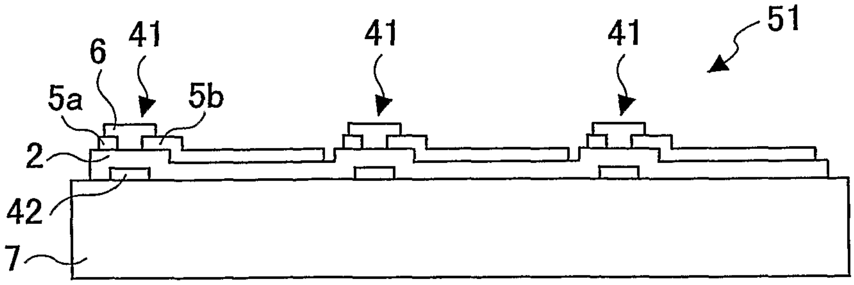


FIG.2B

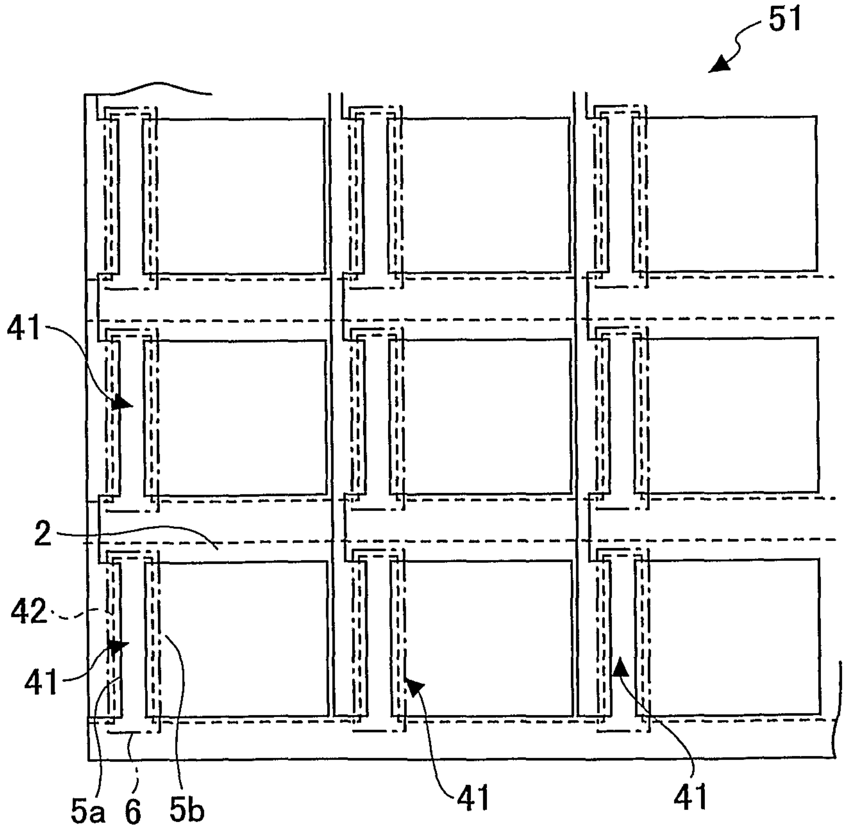

FIG.3

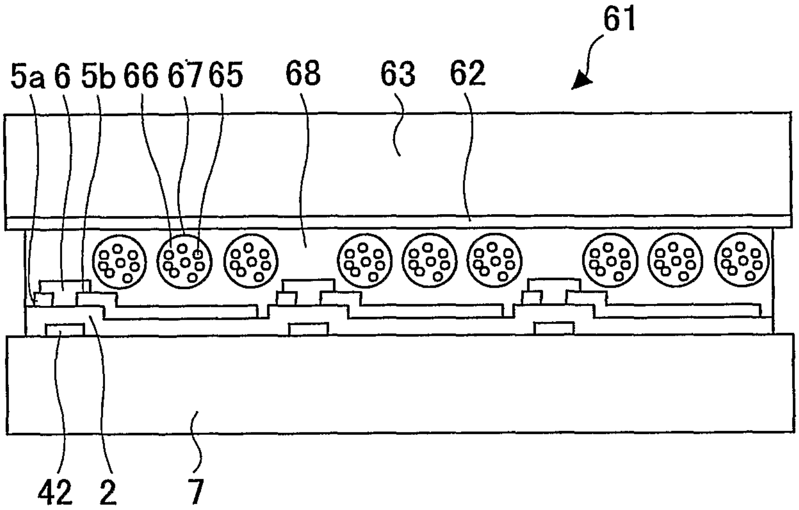


FIG.4

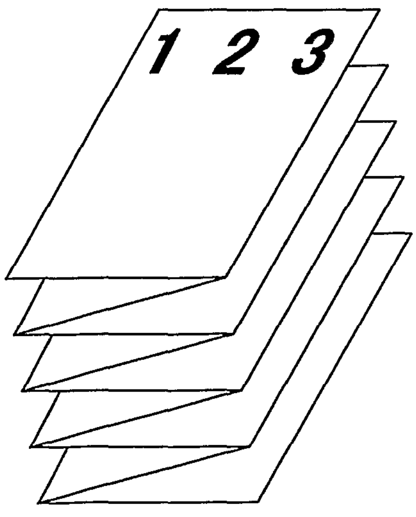

FIG.5

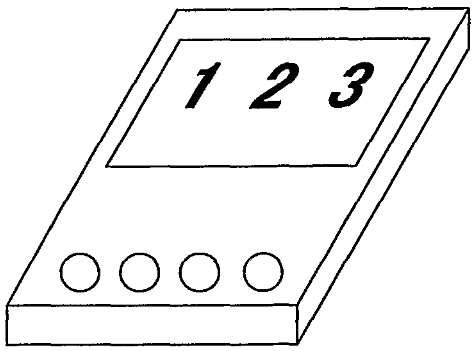

FIG.6

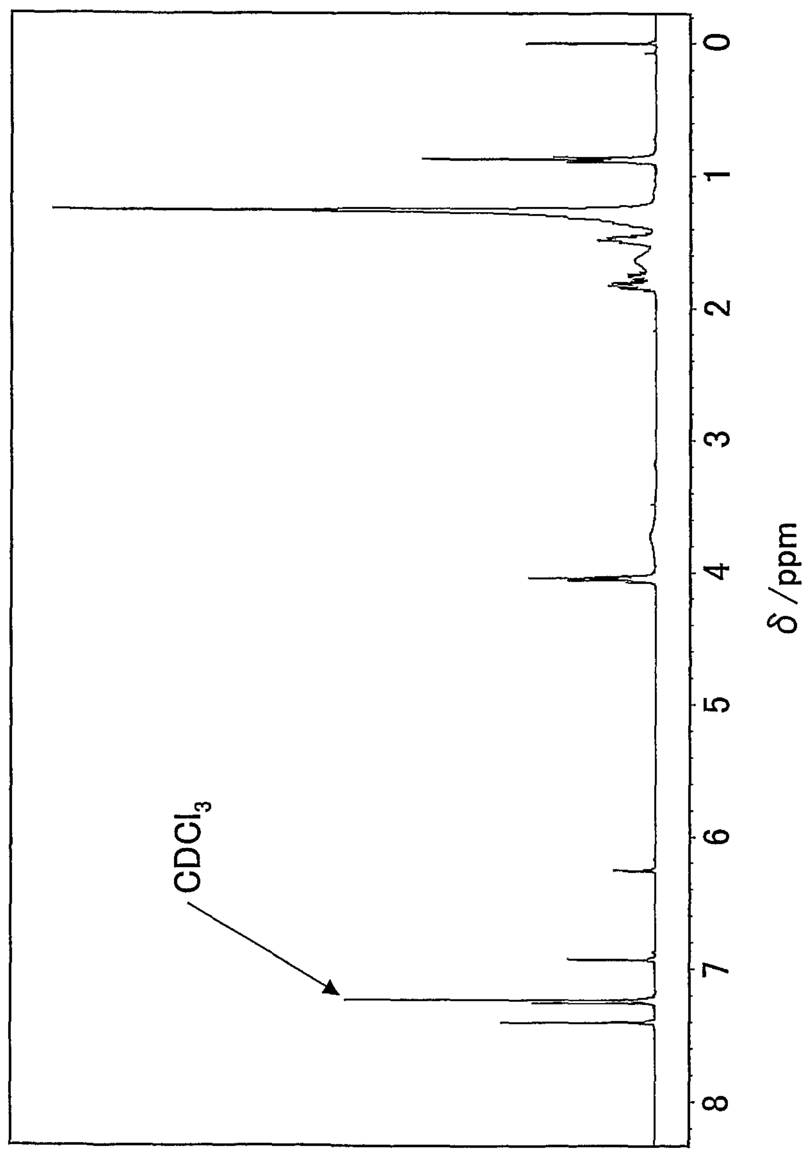

FIG.7

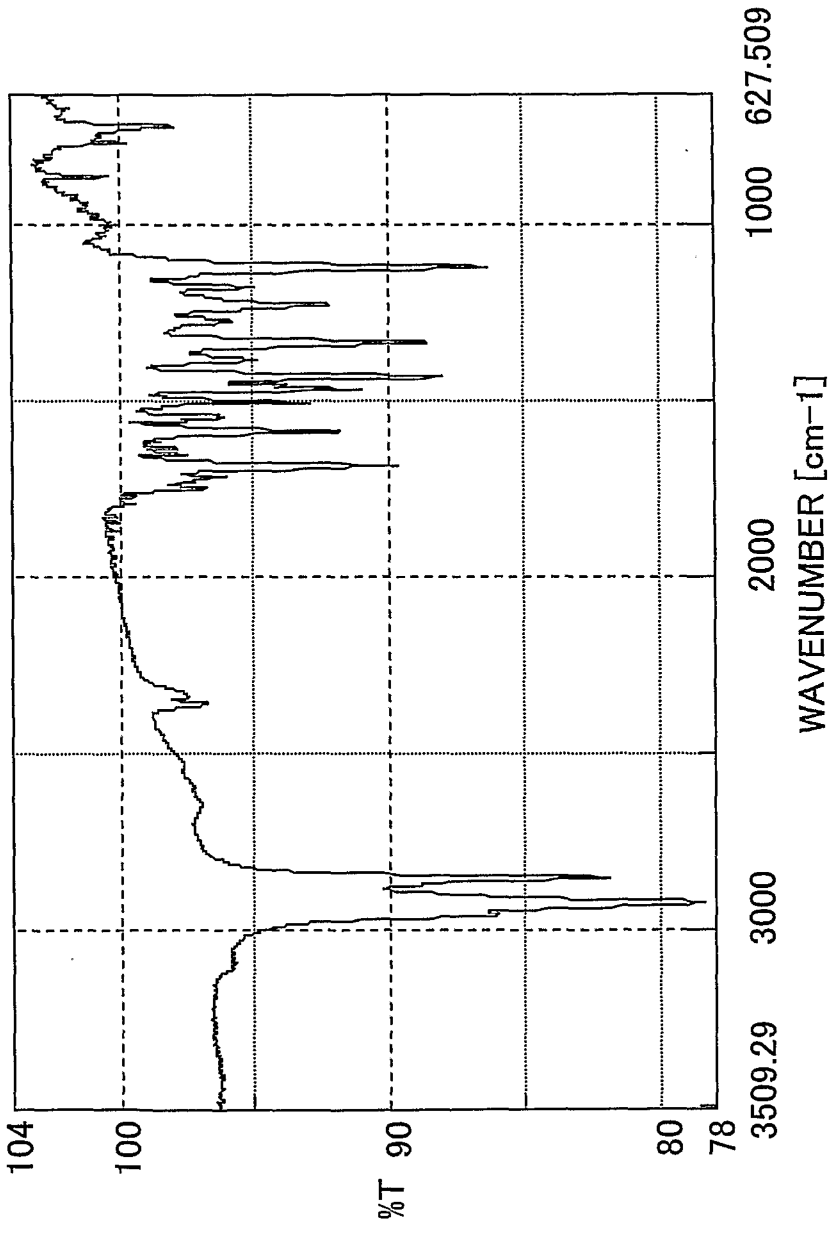
104
100
90
80
78
%T
3509.29
3000
2000
1000
627.509
WAVENUMBER [cm−1]

FIG.8

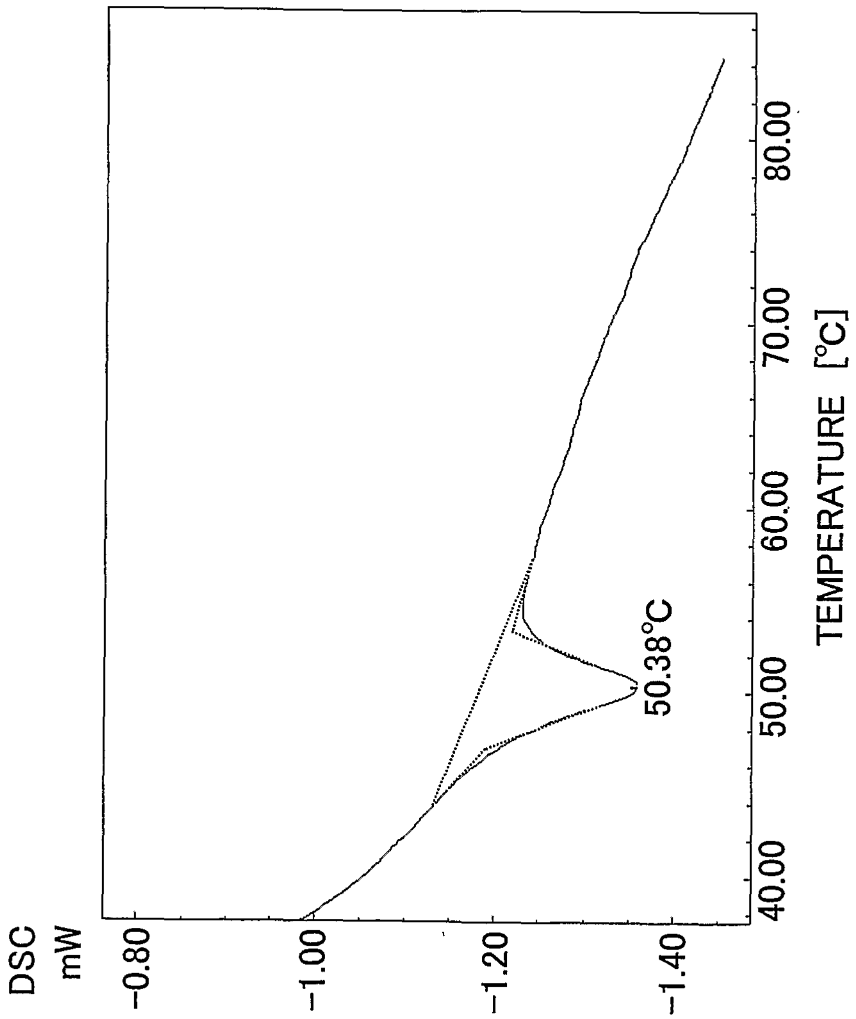
DSC
mW
-0.80
-1.00
-1.20
-1.40
50.38°C
40.00
50.00
60.00
70.00
80.00
TEMPERATURE [°C]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005310962 A
- JP 6345869 A
- US 20060149029 A1
- US 20060073350 A1
- CN 1375538 A
- KR 20000020048 A